# EUROPEAN PATENT APPLICATION

(11) **EP 4 681 710 A1**
(43) Date of publication of application: **21.01.2026**
(21) Application number: 25188034.0
(22) Date of filing: 08.07.2025
(51) Int. Cl.: A61K 31/421, A61K 31/4468, A61P 29/00, A61K 45/06

(54) **2-PENTADECYL-2-OXAZOLINE AS AN OPIOID ADJUVANT TO PREVENT OR TREAT HYPERALGESIA**

(30) Priority: 18.07.2024 IT 202400016696
(71) Applicant: EPITECH GROUP S.p.A., 20144 Milano (MI) (IT)
(72) Inventor: MARCOLONGO, Gabriele, I-20144 Milan (IT); DELLA VALLE, Maria Federica, I-20144 Milano (IT); COLUZZI, Flaminia, I-20144 Milano (IT); DELLA VALLE, Raffaella, I-20144 Milano (IT)
(74) Representative: Long, Giorgio

(57) **Abstract**

The subject of the present invention is the use of 2-Pentadecyl-2-oxazoline (also known as palmitoylethanolamide oxazoline or PEA-OXA) to counteract hyperalgesia induced by the most common opioids (OIH) associated with microglial hyperactivation.

In particular, the present invention concerns the use of PEA-OXA in humans and animals to prevent or treat some of the adverse effects of opioids by controlling immune hyperactivation, wherein said PEA-OXA is administered separately, sequentially or jointly with the opioid.

## Description

### Technical field of the invention

The subject of the present invention is a formulation containing PEA-OXA to counteract, in humans and animals, opioid-induced hyperalgesia (OIH) by controlling microglial hyperactivation and the hyperactivation of other immune cells.

### Prior art

Opioid analgesics are widely used in the management of acute and chronic pain, although their use has long been associated with potential abuse and other serious problems that still limit their use today.

Among the most significant complications, the continuous use of opioids can, paradoxically, worsen rather than reduce pain.

The condition in which the administration of an opioid causes an increase in pain sensitivity is defined as opioid-induced hyperalgesia (OIH).

Identifying and counteracting OIH is of fundamental importance because, at the clinical level, it may be confused with opioid tolerance or with a worsening of the ongoing disease, leading to the inappropriate increase of dosages and consequently to poor pain management. In fact, OIH cannot be resolved by increasing the dosage (as occurs in the case of tolerance), and increasing the dosage may accelerate the onset of tolerance.

Unfortunately, OIH is a phenomenon that does not occur only after chronic opioid treatment. It may in fact arise in the case of acute treatments, for example after surgical procedures.

From a chemical point of view, opioids are alkaloids and may be of natural origin (morphine, codeine and thebaine, also called opiates as they are derived from opium), semi-synthetic (heroin, oxycodone and hydrocodone), or fully synthetic (fentanyl, pethidine, levorphanol, methadone, tramadol and dextropropoxyphene).

Among the synthetic opioids, fentanyl, an analgesic with potency and toxicity at least one hundred times higher than that of morphine, is used, together with its analogues, as an anaesthetic and analgesic both in human and veterinary medicine.

In Italy, fentanyl is prescribed as a general anaesthetic in major surgical procedures and in palliative therapy for terminal cancer pain.

To date, the World Health Organization (WHO) has included this drug in the list of essential medicines for pain in patients with advanced cancer, while its use for the treatment of noncancer chronic pain is still the subject of controversy.

The remaining concerns about the use of opioids such as fentanyl are certainly related to the onset of numerous side effects. In particular, like other opioids, fentanyl induces OIH in humans and companion animals.

The pathophysiological mechanisms underlying OIH have not yet been fully clarified; however, recent evidence highlights the role played by central and peripheral immune cells in the onset of OIH and other adverse effects of opioids. In particular, fentanyl can activate immune cells using the receptors and pathways of pro-inflammatory agents, including the Toll-like receptor 4/myeloid differentiation factor 2 complex (TLR4/MD-2). Hyperactivation of this pathway contributes to exacerbating pro-inflammatory and pronociceptive processes and to promoting the onset of OIH. Among the inflammatory stimuli that activate TLR4/MD-2 is lipopolysaccharide (LPS), a bacterial endotoxin. Opioids, by interacting with TLR4/MD-2, intensify the inflammatory response induced by LPS both in vitro and in vivo. These results have been confirmed by molecular docking studies showing that natural and synthetic opioids bind the TLR4/MD-2 complex through interaction with LPS on MD-2.

In clinical practice, to counteract OIH, opioid antagonist molecules are used, among them nalorphine and naltrexone, or more commonly naloxone. It is interesting to note that naloxone is a non-competitive antagonist of TLR4 and acts by blocking the dimerization of the TLR4/MD-2 complex. It is precisely in this manner that naloxone reduces the appearance of the main side effects of opioids, including OIH. Naloxone is a narcotic that acts on the central nervous system and is not free from significant side effects such as anxiety, disorientation, confusion and hallucinations, aggression, nausea, vomiting, diarrhoea, abdominal pain, rhinorrhoea and, in the most severe cases, pulmonary depression.

For these reasons, there remains an urgent need to develop new therapies to manage, in a more effective and safer way, the side effects of opioids. In this context, the ideal would be the use of molecules that interfere with the TLR4/MD-2 pathway, like naloxone, but that are free from further side effects.

The inventors of the present patent application have surprisingly found that palmitoylethanolamide oxazoline or PEA-OXA (hereinafter referred to as PEA-OXA), is active in counteracting the onset of OIH without presenting the side effects of naloxone.

PEA-OXA recapitulates many of the protective effects of its main metabolite, palmitoylethanolamide (PEA): it presents, in fact, the same safety profile but is characterised by interesting intrinsic properties, not common to PEA. In particular, it has been demonstrated that PEA-OXA, like naloxone, antagonises the dimerization of the TLR4/MD-2 complex thus inhibiting microglial activation. Recently, using an engineered HEK-Blue cell line, characterised by the presence of all the elements of the TLR4 pathway, it has been shown that PEA, unlike PEA-OXA, does not interact with the TLR4/MD-2 pathway and that its protective effects are not mediated by the interaction with the TLR4 receptor but by other mechanisms.

Taken together, these results suggest that PEA-OXA may represent the ideal candidate to be used as an opioid adjuvant, to counteract, in a safe and effective manner, the OIH induced by the opioid fentanyl and any other side effects of opioids that may depend on the TLR4/MD-2 complex.

### Summary of the invention

The present invention derives from the surprising discovery that PEA-OXA interferes with the action of fentanyl on the TLR4/MD-2 complex, an action responsible for immune hyperactivation and onset of OIH.

The invention therefore involves an increase in the safety profile of the patient, both in terms of lower incidence and severity of side effects, in particular OIH, typical of this opioid.

An object of the invention is therefore PEA-OXA for use as an opioid adjuvant to prevent or treat the onset of OIH, wherein PEA-OXA is administered in association or in combination with opioids, in particular fentanyl, wherein said administration is separate, joint or simultaneous.

A further object of the invention is PEA-OXA for use in the treatment of OIH.

A further object of the invention is a composition containing PEA-OXA and an opioid, preferably fentanyl, in particular for use in the prevention of the onset of OIH.

These and further objects, as outlined in the appended claims, will be described in the following description. The text of the claims is to be considered included in the description for the purpose of assessing sufficiency of disclosure.

Further features and advantages of the invention will emerge from the description below of preferred embodiments, provided by way of example and not limitation.

### Brief description of the Figures

Figure 1 shows the experimental scheme of the in vivo study. Fig. 1A: preventive scheme, Fig. 1B: treatment scheme;
Figure 2 shows the effects of treatment with PEA-OXA 30 µM on the reduction of the release of the pro-inflammatory cytokine IL-1β induced by the exposure of microglial cells to LPS + fentanyl and resulting from microglial hyperactivation by fentanyl. ***p < 0.001 LPS vs CTR; °°°p < 0.001 LPS + F vs LPS; ###p < 0.001 LPS + F + PEA-OXA vs LPS + F.
Figure 3 shows the effects of treatment with PEA-OXA 30 µM on the reduction of expression levels of IL-1β and IL-6 genes quantified in hyperactivated microglia following the exposure of cells to LPS + fentanyl:
   A) IL-1β: ***p < 0.001 LPS vs CTR; °°p < 0.01 LPS + F vs LPS; ###p < 0.001 LPS + F + PEA-OXA vs LPS + F.
   B) IL-6: ***p < 0.001 LPS vs CTR; °p < 0.05 LPS + F vs LPS; ###p < 0.001 LPS + F + PEA-OXA vs LPS + F.

### Detailed description of the invention

The present invention concerns in a first aspect PEA-OXA for use as an opioid adjuvant to prevent or treat the onset of OIH, wherein PEA-OXA is administered in association or in combination with opioids, wherein said administration is separate, joint or simultaneous.

The terms "in association" or "in combination" mean both a combination therapy and a therapy wherein PEA-OXA and the opioid are contained in a single dosage form.

By "separate" administration is meant an administration of PEA-OXA and the opioid, administered at different times which may range from 1 minute to several hours, for example 8, 12 or 14 hours apart from each other.

In particular, by "use as an opioid adjuvant to prevent the onset of OIH" is meant an administration of PEA-OXA at a time prior to the administration of the opioid.

By "joint" administration is meant an administration of PEA-OXA and the opioid contained in a single dosage form, i.e. a pharmaceutical or veterinary composition or formulation.

By "simultaneous" administration is meant an administration of PEA-OXA and the opioid in separate dosage forms, but administered simultaneously, that is, within a time separation between the administration of PEA-OXA and that of the opioid, or vice versa, not exceeding 1 minute.

A further object of the invention is PEA-OXA for use in the treatment of OIH induced by opioids.

In the present invention, the opioids are selected from natural opioids or opiates, such as morphine, codeine and thebaine, semi-synthetic opioids, such as heroin, oxycodone and hydrocodone, or synthetic opioids, such as fentanyl, pethidine, levorphanol, methadone, tramadol and dextropropoxyphene. Preferably, the opioid is fentanyl.

A further object of the present invention is a composition comprising PEA-OXA and an opioid. Preferably, the composition of the invention consists of a dry blend of PEA-OXA/opioid.

Whether they are administered separately or jointly in a single formulation, PEA-OXA and the opioid are administered at a PEA-OXA/opioid weight ratio between 100:1 and 600:1, preferably between 150:1 and 500:1.

Based on these weight ratios, for which a significant effect of inhibition of OIH has been observed, the minimum daily dose of PEA-OXA, both in a combination therapy and in a PEA-OXA/opioid composition, shall be at least between 1 mg/day and 500 mg/day.

Such doses may vary depending on the subject and in particular if the subject is of paediatric, adult or elderly age.

It will however be possible to use doses of PEA-OXA higher than those described above, which have proven sufficient to achieve an inhibitory effect on OIH.

Therefore, the overall daily dose of PEA-OXA administered to a subject, both in the form of combination therapy and in said composition with an opioid or for treatment after the onset of OIH, may range between 200 and 2000 mg/day, preferably between 300 and 1500 mg/day or between 400 and 1200 mg/day.

Such daily doses may be divided into unit doses for administration, for example, from 1 to 4 times per day. The dose will also depend on the chosen route of administration. It must be considered that dosage variations may be necessary depending on the age and weight of the patient and also on the severity of the OIH to be treated. The exact dose and route of administration will ultimately be at the discretion of the attending physician.

For the purposes of the invention, PEA-OXA alone, the opioid alone or the composition containing PEA-OXA and opioid may be included in pharmaceutical or veterinary formulations and may be formulated in dosage forms for oral, buccal, parenteral, rectal, topical or transdermal administration.

For oral administration, the compounds of the invention may be, for example, in the form of tablets or capsules, hard or soft, prepared in the conventional manner with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium hydrogen phosphate); lubricants (e.g. magnesium stearate, talc or silica); disintegrants (e.g. potato starch or sodium starch glycolate); or inhibiting agents (e.g. sodium lauryl sulphate). The tablets may be coated using methods well known in the art. Liquid preparations for oral administration may be in the form of solutions, syrups or suspensions or may be presented as lyophilised products or granules to be reconstituted, prior to use, with water or other suitable vehicles. Such liquid preparations may be prepared by conventional methods with pharmaceutically acceptable additives such as suspension agents (e.g. sorbitol syrup, cellulose derivatives or edible hydrogenated fats); emulsifying agents (e.g. lecithin or acacia); non-aqueous vehicles (e.g. almond oil, oily esters, ethyl alcohol or fractionated vegetable oils); and preservatives (e.g. methyl- or propyl-p-hydroxybenzoates, sorbic acid, benzoic acid or their salts). The preparation may also suitably contain flavourings, colourings and sweeteners.

Preparations for oral administration may be suitably formulated to allow controlled release of the active ingredient.

For buccal administration, the compounds of the invention may be in the form of tablets or granules formulated in the conventional manner, suitable for absorption at the buccal mucosa level. Typical buccal formulations are sublingual tablets.

The compounds of the invention may be formulated for parenteral administration by injection. Injection formulations may be presented in single dose form, for example in ampoules, with an added preservative. The compositions may be in this form as suspensions, solutions or emulsions in oily or aqueous vehicles and may contain formulation agents such as suspending, stabilising and/or dispersing agents. Alternatively, the active ingredient or the mixture of active ingredients may be in the form of a powder to be reconstituted, prior to use, with a suitable vehicle, for example with sterile water.

The compounds of the invention may also be formulated in rectal formulations such as suppositories or retention enemas, for example containing the basic components of common suppositories such as cocoa butter or other glycerides.

In addition to the formulations described above, the compounds of the invention may also be formulated as depot preparations for administration over a period ranging from one day to one week. Such long-acting formulations may be administered by implantation (e.g. subcutaneously, transcutaneously or intramuscularly) or by intramuscular injection. Thus, for example, the composition may be formulated with appropriate polymeric or hydrophobic materials (for example in the form of an emulsion in a suitable oil) or ion exchange resins or as minimally soluble derivatives.

The compounds or the composition of the invention may also be administered in the form of oral sprays or nasal sprays.

A further object of the invention also includes dietary compositions, dietary supplements, complementary feeds and foods for special medical purposes (FSMP) comprising PEA-OXA.

The term "foods for special medical purposes" refers to products authorised according to Regulation (EU) 2016/128. This term refers to a product to be administered under medical supervision, thus assimilating said FSMP to a medicinal product.

The formulations according to the invention may be prepared by conventional methods, such as those described in Remington's Pharmaceutical Sciences Handbook, Mack Pub. Co., N.Y., USA, 17th edition, 1985 or in Remington, The Science and Practice of Pharmacy, Edited by Allen, Loyd V., Jr, 22nd edition, 2012 or subsequent editions.

### Experimental part

### Biological testing

To evaluate the effect of PEA-OXA on microglial hyperactivation induced by the opioid fentanyl, primary microglial cells were obtained from cerebral cortices of neonatal rats (Sprague-Dawley) aged 1-2 days (Facci L. et al, Astrocyte/microglia cocultures as a model to study neuroinflammation, Methods Mol. Biol. 2018; 1727: 127-137).

Microglia were exposed to fentanyl (F, 10 µM) and to LPS (10 ng/ml), in the absence or presence of PEA-OXA (30 µM) for 24h. At the end of the treatments, the culture medium was collected and used to quantify the pro-inflammatory interleukin (IL)-1β using the ELISA technique with a commercially available kit (Antigenix America, Huntington Station, NY, USA). The absorbance of each sample was measured at 450 nm and the concentration of IL-1β was determined by referring to a standard curve obtained with known amounts of IL-1β (Bisceglia F. et al, Prenylated curcumin analogues as multipotent tools to tackle Alzheimer's disease, ACS Chem. Neurosci. 2019; 10: 1420-1433).

Gene transcript analyses were conducted using the Real Time-PCR (RT-PCR) technique to quantify the expression of IL-1β and IL-6 genes. Briefly, total RNA was extracted from microglial cells using the TRIzol reagent (Invitrogen). The quality and quantity of the extracted RNA were determined using the Agilent RNA 6000 Nano Kit instrument (Agilent Technologies). The reverse transcription of RNA into complementary DNA (cDNA) was performed using the Superscript III reverse transcriptase (Invitrogen). RT-PCR was performed as described in Barbierato, M. et al, Astrocyte-microglia cooperation in the expression of a pro-inflammatory phenotype, CNS Neurol. Disord. Drug Targets 2013; 12: 608-618.

For the in vivo study, C57BL/6 mice approximately three months old were used to evaluate the effectiveness of PEA-OXA in reducing OIH induced by the opioid fentanyl.

Before the start of the study, the animals underwent a one-week acclimation period at the animal facility of the University of Padua, following all procedures and experimental protocols in accordance with the principles of care and welfare of laboratory animals approved by the Italian Ministry of Health (Legislative Decree 2014/26), European Directives (EU Directive 2010/63), and the ARRIVE guidelines.

To induce OIH, the protocol described in Chen D. et al, The blockade of neuropeptide FF receptor 1 and 2 differentially contributed to the modulating effects on fentanyl-induced analgesia and hyperalgesia in mice, Eur J Pharmacol. 2024; 969: 176457 was used.

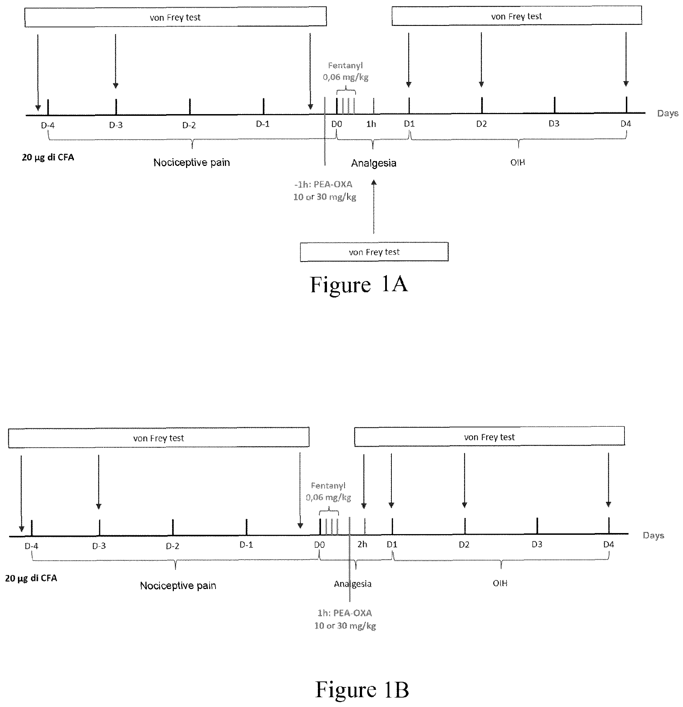

Before the beginning of the experiment, the von Frey test was performed at baseline to measure the animals' pain threshold. Four days before the administration of fentanyl, 20 µl containing 1 mg/ml of complete Freund's adjuvant (CFA) (Sigma-Aldrich) were injected into the hind paw of the animals to simulate a nociceptive insult. The von Frey test was repeated the day after the injection with CFA (D-3). Four days later (D0), the animals were subjected to the von Frey behavioural test before pre-treatment with PEA-OXA (10 or 30 mg/kg). Fentanyl, administered as four subcutaneous injections (s.c., 4 x 60 µg/kg) at 15-minute intervals, was given 1 hour after PEA-OXA. To assess the development of OIH, the von Frey test was repeated 1h, 1, 2 and 4 days after the administration of fentanyl (Fig. 1A, preventive scheme).

Similarly, a second group of animals, four days after the administration of LPS (D0), was subjected to the von Frey behavioural test 1h before the administration of the four s.c. injections of fentanyl. Treatment with PEA-OXA (10 or 30 mg/kg) was administered 1 hour after the opioid. To assess the development of OIH, the von Frey test was repeated 2h, 1, 2 and 4 days after the administration of fentanyl (Fig. 1B, treatment scheme).

Control animals were subjected to the same procedures, injecting the same volumes of the vehicles of the substances used.

As described above, all animals were subjected to the von Frey test, a behavioural test that assesses the pain threshold in animals.

The mice were placed inside a box equipped with a metal mesh floor, and mechanical sensitivity was assessed in the mid-plantar area of each hind paw by means of a mechanical stimulus consisting in the application of a filament (Ugo Basile) delivering a force ranging from 0 to 5 g.

Gradual and increasing stimuli from beneath the mesh were applied at 5-second intervals using a metal stylus.

Latency was defined as the maximum force causing paw withdrawal (Chen D et al., 2024, above).

### Statistical analysis

Statistical analyses were carried out using GraphPad software, version 3.03 (GraphPad Software Inc., La Jolla, CA, United States). The results of the studies were expressed as mean ± standard error of the mean (SEM).

Statistical differences in the in vitro study were analysed using one-way ANOVA followed by Holm-Sidak's post-hoc test, whereas the results of the in vivo study were analysed using two-way ANOVA followed by an appropriate post-hoc test for multiple comparisons. A p-value < 0.05 was considered significant.

### Results of the experiments

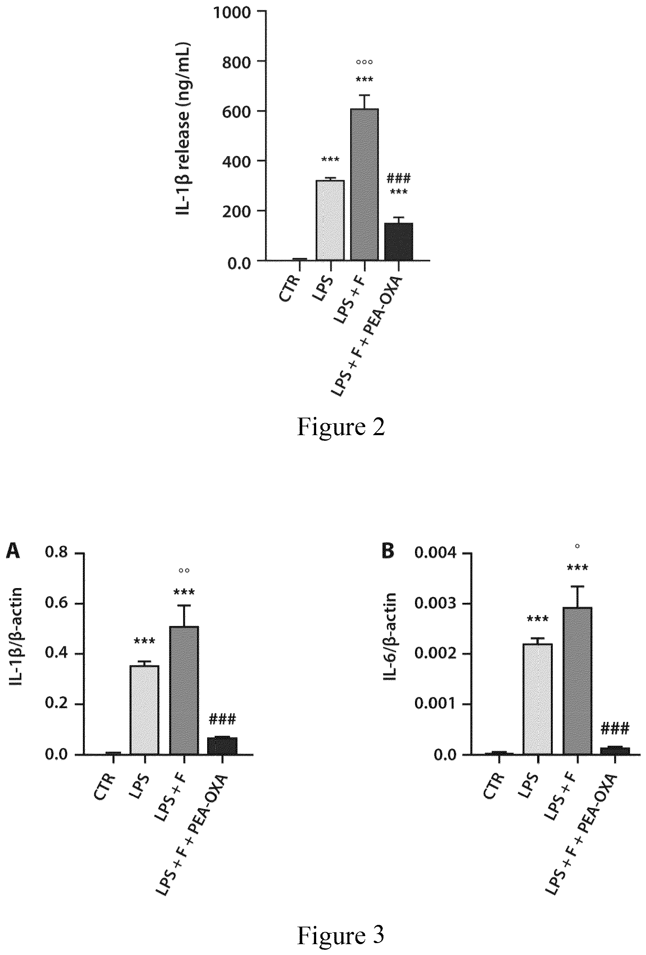

As shown in Fig. 2, the exposure of microglial cells to LPS induces a marked increase in the release of IL-1β (***p < 0.001 LPS vs CTR). The exposure of microglia to LPS + fentanyl further increases the release of IL-1β (°°°p < 0.001 LPS + F vs LPS), indicative of microglial cell hyperactivation. Treatment with PEA-OXA 30 µM significantly reduces microglial hyperactivation induced by stimulation with LPS + F. Specifically, IL-1β levels released after PEA-OXA 30 µM are approximately 180 ng/ml vs 600 ng/ml released in the absence of treatment (Fig. 1, ###p < 0.001 LPS + F + PEA-OXA vs LPS + F).

As shown in Fig. 3, the exposure of microglial cells to LPS induces a marked increase in the expression of IL-1β (Fig. 3A) and IL-6 (Fig. 3B) genes (***p < 0.001 LPS vs CTR). The exposure of microglia to LPS + fentanyl further increases the expression of IL-1β (°°p < 0.01 LPS + F vs LPS) and IL-6 (°p < 0.05 LPS + F vs LPS) genes, hyperactivating the microglial cells. Treatment with PEA-OXA 30 µM significantly reduces gene transcripts in microglia hyperactivated by LPS + F treatment. Specifically, the expression of IL-1β and IL-6 genes is significantly reduced by pre-treatment with PEA-OXA 30 µM compared to untreated microglia (###p < 0.001 LPS + F + PEA-OXA vs LPS + F).

The invention will now be further described by means of the following formulation examples.

### Formulation Examples

PEA-OXA = 2-pentadecyl-2-oxazoline

### Example 1 - Hard capsule for human use

Acid-resistant vegetable gelatin capsule, size "0"

| | |
|---|---|
| PEA-OXA | 300 mg |
| Maize Dextrin | 100 mg |
| Silicon Dioxide | 10 mg |
| Magnesium Stearate | 10 mg |

### Example 2 - Gastro-resistant tablet

| | |
|---|---|
| PEA-OXA | 400 mg |
| Dextrose | 52 mg |
| Rice starch | 120 mg |
| Microcrystalline cellulose | 120 mg |
| Polysorbate 80 of vegetable origin | 8 mg |
| Silicon Dioxide | 10 mg |
| Gastro-resistant coating | 40 mg |

### Example 3 - Orodispersible granules

| | |
|---|---|
| PEA-OXA | 400 mg |
| Glyceryl Palmitostearate | 100 mg |
| Sorbitol | 180 mg |
| Fructose | 180 mg |
| Flavouring | 50 mg |
| Polysorbate 80 | 10 mg |
| PVP K30 | 10 mg |
| Sucrose Palmitate | 20 mg |

### Example 4 - Suppositories of 2.0 g

| | |
|---|---|
| Suppository base (C10-18 Triglycerides-Polysorbate 65) | 1695 mg |
| PEA-OXA | 200 mg |

### Example 5 - Soft capsule for human use

| | |
|---|---|
| Bovine Gelatine | 237 mg |
| PEA-OXA | 200 mg |
| Glycerol | 129 mg |
| Sunflower Oil | 366 mg |
| Medium Chain Triglycerides | 51.5 mg |
| Vegetable Fat | 50.8 mg |
| Soy Lecithin | 30.0 mg |
| Water | 18.8 mg |
| Glyceryl Monostearate | 8.8 mg |

### Example 6 - Single-dose injectable solution

| | |
|---|---|
| PEA-OXA | 10 mg |
| Soy Lipids | 200 mg |
| Egg Lecithin | 24 mg |
| Glycerol | 100 mg |
| Water for injections to | 2 ml |

### Example 7 - Nasal spray 10 ml multidose bottle / dose 0.15 ml

| | |
|---|---|
| Methyl-beta-cyclodextrin | 1000 mg |
| Glycerol | 85 mg |
| Disodium Phosphate Dodecahydrate | 28 mg |
| Tri potassium Citrate Monohydrate | 25 mg |
| Hyaluronic Acid Sodium Salt | 7.5 mg |
| Monosodium Phosphate Dihydrate | 6.3 mg |
| PEA-OXA | 5 mg |
| Sodium Chloride | 3 mg |
| Citric Acid Monohydrate | 0.19 mg |
| Water for injections to | 10 ml |

### Example 8 - Lyophilised powder for nasal inhalation

| | |
|---|---|
| PEA-OXA | 0.3 mg |
| Methyl-beta-cyclodextrin | 68 mg |
| Tripotassium Citrate | 2 mg |
| Lactose | 29.7 mg |
| Hard gelatin capsule, size 4. | |

## Claims

1. 2-Pentadecyl-2-oxazoline (PEA-OXA) for use as an opioid adjuvant to prevent the onset of or in the treatment of opioid-induced hyperalgesia (OIH), wherein PEA-OXA is administered in association or in combination with opioids, wherein said administration is separate, joint or simultaneous.

2. 2-Pentadecyl-2-oxazoline for use according to claim 1, wherein the opioids are selected from natural opioids or opiates, such as morphine, codeine and thebaine, semi-synthetic opioids, such as heroin, oxycodone and hydrocodone, or synthetic opioids, such as fentanyl, pethidine, levorphanol, methadone, tramadol and dextropropoxyphene.

3. 2-Pentadecyl-2-oxazoline for use according to claim 2, wherein the opioid is fentanyl.

4. 2-Pentadecyl-2-oxazoline for use according to any one of claims 1 to 3, wherein 2-pentadecyl-2-oxazoline and opioid are administered at a PEA-OXA/opioid weight ratio between 100:1 and 600:1, preferably between 150:1 and 500:1.

5. 2-Pentadecyl-2-oxazoline for use according to any one of claims 1 to 4, wherein the minimum daily dose of 2-pentadecyl-2-oxazoline, both in a combination therapy and in a PEA-OXA/opioid composition, is at least between 1 mg/day and 500 mg/day.

6. 2-Pentadecyl-2-oxazoline for use according to any one of claims 1 to 5, wherein the overall daily dose of PEA-OXA administered to a subject, both in the form of combination therapy and in said composition with an opioid or for treatment after the onset of OIH, is between 200 and 2000 mg/day, preferably between 300 and 1500 mg/day or between 400 and 1200 mg/day.

7. A pharmaceutical or veterinary composition comprising 2-pentadecyl-2-oxazoline, optionally an opioid, wherein the opioid is preferably fentanyl, for use in the treatment or prevention of opioid-induced hyperalgesia.

8. 2-Pentadecyl-2-oxazoline for use as an opioid adjuvant to prevent the onset of or in the treatment of OIH, wherein 2-pentadecyl-2-oxazoline is contained in a dietary composition, a dietary supplement, a complementary feed, or a food for special medical purposes (FSMP).
